(19) Europäisches Patentamt
European Patent Office
Office européen des brevets

(11) Publication number: **0 150 952**
**B1**

(12) **EUROPEAN PATENT SPECIFICATION**

(45) Date of publication of patent specification: **30.08.89**

(51) Int. Cl.$^4$: **C 08 F 2/50,** G 03 C 1/14, A 61 K 7/06

(21) Application number: **85300264.0**

(22) Date of filing: **15.01.85**

(54) **Photopolymerizable composition.**

(30) Priority: **17.01.84 JP 6797/84**

(43) Date of publication of application:
**07.08.85 Bulletin 85/32**

(45) Publication of the grant of the patent:
**30.08.89 Bulletin 89/35**

(84) Designated Contracting States:
**DE FR GB IT NL**

(56) References cited:
**US-A-3 368 900**

(73) Proprietor: **KURARAY CO., LTD.**
**1621 Sakazu**
**Kurashiki-City Okayama Prefecture 710 (JP)**

(72) Inventor: **Hino, Kenichi**
**1660, Sakazu**
**Kurashiki-city (JP)**
Inventor: **Yamauchi, Junichi**
**21-7, Hachiojicho**
**Kurashiki-city (JP)**
Inventor: **Nishida, Koji**
**c/o Ranpuryo, 1652-1**
**Sakazu Kuranshiki-city (JP)**

(74) Representative: **Crampton, Keith John Allen et al**
**D YOUNG & CO 10 Staple Inn**
**London WC1V 7RD (GB)**

EP 0 150 952 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to photopolymerizable compositions, especially for use in dental applications, and is more particularly concerned with polymerization initiators contained in such compositions.

Photopolymerizable resins which cure upon exposure to ultraviolet rays and visible light have recently been used to replace thermosetting resins in many applications such as paints, printing inks, adhesives, coating materials, and dental filling materials.

Hitherto photopolymerizable dental composite resins have been of UV-curable type or visible-light-curable type. Those of UV-curable type have the disadvantage that curing by irradiation does not reach as deeply as required so that a part of the resin remains uncured at the innermost recess of the tooth cavity. Moreover, it has poor adhesion to the tooth. These disadvantages have been overcome to some extent with the advent of visible-light-curable composite resins. Nevertheless, they are not cured completely when a deep tooth cavity is filled in a single application, and filing has to be carried out twice. It is considered that these drawbacks result from the insufficient curing performance of the photopolymerization initiator.

Conventional polymerization initiators for photopolymerizable compositions are disclosed in U.S. Patents Nos. US—A—4,071,424 and US—A—4,459,193. The former discloses the use of an α-diketone and an amine as the photosensitizer and accelerator, and the latter teaches the combined use of fluorenone or an α-diketone and an organic peroxide, optionally with an amine. These conventional polymerization initiators have some disadvantages. The α-diketone/amine types and the (fluorenone or α-diketone)/organic peroxide types are not sufficient in curing rate and cure depth, and consequently are not of practical use. On the other hand, the (fluorenone or α-diketone)/organic peroxide/amine types have a high curing rate but are so unstable in storage that they cannot be packaged together with a resin composition. The poor storage stability may be overcome by packaging the amine separately from the (α-diketone or fluorenone)/peroxide and combining the separate packs just before use, but this is troublesome and undesirable.

Japanese Laid-Open Specification No. 130693/1979 discloses a UV polymerization initiator composed of benzil and N,N-dialkylaminobenzaldehyde. This is not of practical use either because of its poor storage stability and its tendency to discolour the cured product.

US Patent No. US—A—3,368,900 discloses photopolymerisable compositions based e.g. on (meth)acrylate ester monomers, and including a polynuclear quinone photoinitiator and an aromatic aldehyde.

The present invention provides a photopolymerizable composition composed of a polymerizable monomer and an initiator capable of polymerizing the said monomer upon exposure to visible light, in which the initiator consists essentially of (a) at least one photosensitizer that is an aliphatic or cycloaliphatic α-diketone, (b) at least one accelerator selected from aldehydes containing no amino groups and (c) an organic peroxide.

By proceeding in accordance with preferred embodiments of the invention, it is possible to produce a composition containing a photopolymerization initiator that cures at a high rate of a sufficient depth, that hardly discolours and that has good storage stability.

Compositions in accordance with the present invention are used in restoring tooth cavities.

The polymerizable monomer is selected depending on the desired application; it is usually a (meth)acrylic ester for dental use. Other preferred examples include esters of α-cyanoacrylic acid, crotonic acid, cinnamic acid, sorbic acid, maleic acid, and itaconic acid with a monohydric or dihydric alcohol; (meth)acrylamides such as N-isobutylacrylamide; carboxylic vinyl esters such as vinyl acetate; vinyl ethers such as butyl vinyl ether; mono-N-vinyl compounds such as N-vinyl-2-pyrolidone; and styrene and its derivatives.

Preferred examples of the (meth)acrylic ester are mono- and polyfunctional esters and urethane(meth)acrylate as listed below.

(i) Monofunctional:

Methyl (meth)acrylate, n- or i-propyl (meth)acrylate, n-, i-, or t-butyl(meth)acrylate, 2-hydroxyethyl methacrylate.

(ii) Difunctional:

A di(meth)acrylate of a glycol represented by the formula:

$$CH_2 = \overset{\displaystyle R^1}{\overset{|}{C}} - CO \left( O - CH_2 - CH_2 \right)_n O - CO - \overset{\displaystyle R^1}{\overset{|}{C}} = CH_2$$

wherein n is an integer of from 1 to 14, and $R^1$ is a hydrogen atom or a methyl group, the glycol being e.g. ethylene glycol, diethylene glycol, triethylene glycol, tetraethylene glycol, dodecaethylene glycol or tetradecaethylene glycol; glycerin di(meth)acrylate, 2,2′-bis[p-(γ-methacryloxy-[β-hydroxypropoxy)-phenyl]propane, bisphenol A dimethacrylate, neopentyglycol di(meth)acrylate, 2,2′-di(4-methacryloxy-polyethoxyphenyl)propane with 2 to 10 ethoxy groups per molecule, and 1,2-bis(3-methacryloxy-2-hydroxypropoxy)-butane.

(iii) Tri- or polyfunctional:

Trimethylolpropane tri(meth)acrylate, and pentaerythritol tetra(meth)acrylate.

(iv) Urethane(meth)acrylate:

Reaction products of 2 mols of (meth)acrylate monomer having hydroxyl groups and 1 mol of diisocyanate; and reaction products of a urethane prepolymer with terminal NCO groups and a (meth)acrylate monomer having hydroxyl groups. The reaction product has the formula below:

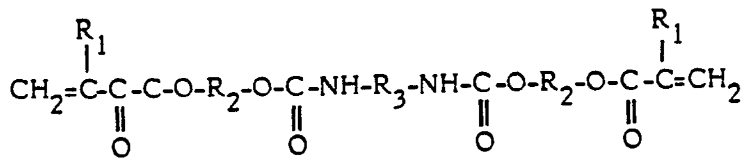

wherein $R_1$ is a hydrogen atom or a methyl group, $R_2$ is an alkylene group, and $R_3$ is an organic residue.

Examples include the reaction product of 2,2,4-trimethylhexamethylene diisocyanate and hydroxypropyl methacrylate as disclosed in Japanese Patent Publication No. 36960/1976; and the reaction product of a urethane prepolymer having terminal isocyanate groups and 2-hydroxyethyl methacrylate as disclosed in Japanese Patent Publication No. 33687/1980. Another monomer that can be used in this invention is a tetrafunctional one as disclosed in U.S. Patent No. US—A—4,386,912 (see Example 3 mentioned later).

In addition to the above-mentioned monomers, it is possible to use a polymerizable monomer having an acid group capable of bonding to teeth and metal and other adherents. Usually, such a monomer is used in a small amount in combination with the above-mentioned monomer. Examples of such monomers include those that contain carboxyl or carboxylic anhydride groups of (meth)acrylic acid, maleic anhydride, crotonic acid, or 4-(meth)acryloxyethyltrimellitic acid; di(meth)acryloxyglycerophosphoric acid, (meth)acryloxyethylphosphoric acid, and glyceroldi(meth)acrylate monofluorophosphate; and those that contain a phosphoric acid or phosphoric chloride group as disclosed in U.S. Patents Nos US—A—4,259,075, US—A—4,259,117, and US—A—4,368,043; European Patent Specifications Nos. EP—A—0 074 708 and EP—A—0 058 483; and Japanese Laid-Open Patent Specification No. 164171/1982.

The photosensitizer used in this invention is at least one aliphatic or cycloaliphatic α-diketone which gives a UV and visible spectrum with apparent absorption at 380 to 500 nm, and that is preferably represented by the formula

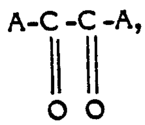

where each A denotes an aliphatic hydrocarbon group of 1 to 20 carbon atoms, which may be the same or different from the other A, or the two As are bonded to one another to form a cycloaliphatic structure.

Examples of these photosensitizers include acyclic α-diketone compounds such as diacetyl, 2,3-pentanedione, 2,3- or 3,4-hexanedione, and 2,3-, 3,4-, or 4,5-octanedione; and alicyclic α-diketone compounds such as camphorquinone (common name of 1,7,7-trimethylbicyclo[2.2.1]heptane-2,3-dione) and bicyclo[2.2.1]heptane-2,3-dione.

Preferably among them are camphorquinone, 2,3-pentanedione, and 2,3-, 3,4- and 4,5-octane-dione. The photosensitizer is used in concentration of 0.01 to 5 wt% based on the quantity of the polymerizable monomer.

The aldehyde used as the accelerator in this invention should preferably be a compound represented by the formula $B{-}(CHO)_n$ (where B denotes an acyclic or alicyclic saturated or unsaturated aliphatic hydrocarbon group having one to 20 carbon atoms or a monocyclic or polycyclic aromatic hydrocarbon group of 1 to 20 carbon atoms; the hydrocarbon group may have a substituent group of 1 to 20 carbon atoms; examples of the substituent group include alkyl groups, polyalkyl ether groups, alkenyl groups, cycloalkyl groups, aryl groups, alkylaryl groups, arylalkyl groups, acyl groups, hydroxyl groups, carboxyl groups, alkoxycarbonyl groups, alkoxy groups, phenoxy groups, halogeno groups, a cyano group, a cyanomethyl group, alkylthio groups, a thiol group, a carbamoyl group, and alkylamide groups, and n is an integer of 1 to 3.) Preferred compounds are alkylmonoaldehydes or alkyldialdehydes of 1 to 20 carbon atoms; polyalkyl ether mono- or dialdehydes of 1 to 20 carbon atoms; and those represented by formula

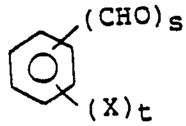

(where s is 1 or 2; X denotes an alkyl, alkoxy, or polyalkylether group of 1 to 20 carbon atoms; and t is an integer of 0 to 3).

Examples of such aldehyde compounds include valeraldehyde, 2-ethylhexylaldehyde, decylaldehyde, tetradecylaldehyde, octadecylaldehyde, citral, citronellal, farnesal, tetrahydrocitral, hexahydrofarnesal, hexahydrobenzaldehyde, phenylacetaldehyde, 2-(p-t-butylbenzyl)propionaldehyde, aldol, hydroxycitronellal, glutaric dialdehyde, 1,9-nonanedial,

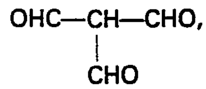

2-methoxyethoxyacetaldehyde ($CH_3$—O—$CH_2CH_2$—O—$CH_2CHO$), 2-(2-methoxyethoxy)ethoxy-acetaldehyde ($CH_3O$—$CH_2CH_2O$—$CH_2CH_2O$—$CH_2CHO$), p-[2-(2-methoxyethoxy)ethoxy]benzaldehyde

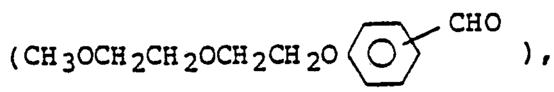

2-(2-methoxyethoxy)ethylbenzaldehyde

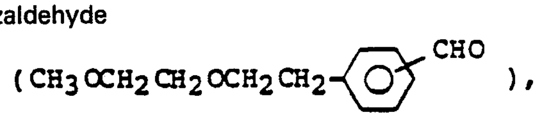

benzaldehyde, (o,m,p)-tolualdehyde, (o,m,p)-octylbenzaldehyde, (o,m,p)-anbisaldehyde, (o,m,p)-butyloxy-benzaldehyde, (o,m,p)-octyloxybenzaldehyde, 3,4-diethoxybenzaldehyde, 3,4,5-trimethoxybenzaldehyde, (o,m,p)-phthalicdicarboaldehyde, (o,m,p)-hydroxybenzaldehyde, (o,m,p)-phenylbenzaldehyde, (o,m,p)-phenoxybenzaldehyde, (o,m,p)-cyanobenzaldehyde, (o,m,p)-chlorobenzaldehyde, 2,4-dichloro-benzaldehyde,

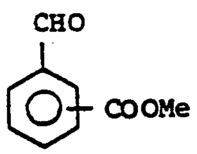 , 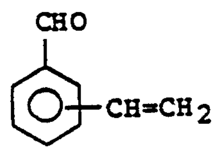 , 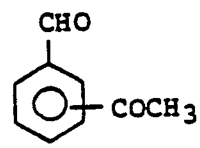 , 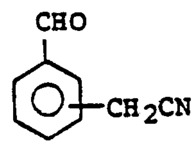 ,

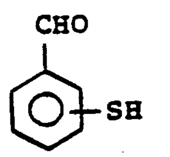 , 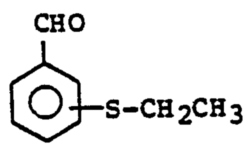 , 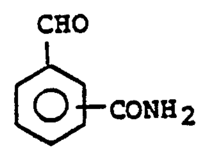 ,

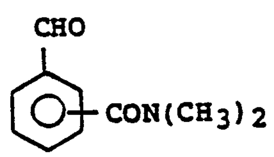 , 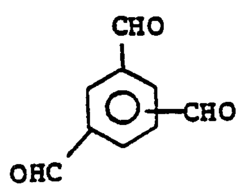 , 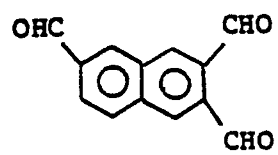

2-naphthoaldehyde, 1,2,3,4-tetrahydro-2-naphthaldehyde, $\Delta^3$-tetrahydrobenzaldehyde, 3,6-dioxaoctane-1,8-dial. Those which have a weak smell or pleasant odor are of practical use.

Those compounds which are not preferable in this invention are low-molecular weight (lower than 80) aliphatic aldehydes such as acetaldehyde and butanal, which are ill-smelling and inflammable. Those aldehydes having an amino group are not used either in this invention, because they are unstable. Although dialkylaminobenzaldehydes are exceptionally stable, they are not suitable for use in this invention because they discolor the cured resin composition when used in combination with an α-diketone.

The above-mentioned aldehyde is used in a concentration of 0.1 to 10 wt% based on the quantity of the polymerizable monomer.

In order to increase the curing rate in this invention, an organic peroxide is added to the photosensitizer and accelerator. In the case of conventional photopolymerization initiators composed of α-diketone and amine, the addition of organic peroxide substantially decreases the storage stability due to the amine-peroxide redox reaction. In contrast, the photopolymerization initiator used in this invention permits the combined use of organic peroxide, ensuring a storage stability longer than one year. This invention provides a photopolymerization initiator which is superior in both curing rate and storage stability. Such performance has never been expected from the prior art.

Examples of the organic peroxide used in this invention include diacyl peroxides and peroxyesters. Preferable among these peroxides are benzoyl peroxide, t-butyl perbenzoate, di-t-butyl diperoxyiso-

phthalate, 2,5-dimethyl-2,5-di(benzoylperoxy)-hexane, and other peroxides, in the form of derivatives of benzoic acid. The peroxide is used in a concentration of 0.1 to 10 wt% based on the quantity of the polymerizable monomer.

The composition of this invention may be used, in addition to the above-mentioned polymerizable monomer and photopolymerization initiator, with various kinds of organic or inorganic fillers. Organic fillers include polymethyl (meth)acrylate and polyethyl (meth)acrylate and polymer-coated inorganic fillers. Inorganic fillers include silicon dioxide, alumina, glass, ceramics, diatomaceous earth, kaolin, montmorillonite, clay, Japanese acid clay, synthetic zeolite, mica, calcium fluoride, calcium phosphate, barium sulfate, zirconium dioxide, titanium dioxide, etc. in the form of powder, fiber, or flake. In the case where the composition of this invention is used in dental applications, the inorganic filler should usually be treated with γ-methacryloxypropopyltrimethoxysilane, vinyltrichlorosilane, vinyltriethoxysilane, vinyltrimethoxysilane, vinyltriacetoxysilane, vinyltri(methoxyethoxy)silane, and other silane compounds. The surface treatment may be accomplished in the usual way.

In the case where the composition of the invention is used as a dental filling compound as mentioned later, the filler should be added in an amount of 20 to 90 wt%, preferably 50 to 85 wt%, based on the composition, and the quantity of the polymerizable monomer should be 80 to 10 wt%, preferably 50 to 15 wt%, based on the composition.

Moreover, the composition of this invention may include a polymerization inhibitor, coloring agent, UV absorber, and other additives, as required.

The above-mentioned components are usually mixed into a paste or liquid by the manufacturer, and it is delivered to the user in one or two light-shielding containers. The user will apply the composition to a substrate and cure it in the usual way by irradiating it with visible light.

As mentioned above, the composition of this invention contains a specific photopolymerization initiator, cures at a high rate, and has good storage stability. The cured product thereof is not discolored. Thus, it greatly out-performs conventional photopolymerizable compositions.

The composition of the invention will find use as dental restorative materials, paints, printing inks, adhesives, films, and other coating materials. It is particularly useful as a dental composite filling material, for filling and restoring tooth cavities. In addition, it will find use as a dental crown material, artificial tooth material, dental adhesive, dental cementing material, and filling material for preventing caries.

In the case where the composition of this invention is used as a dental composite filling material, it is incorporated with a filler. Prior to filling, the tooth cavity is etched with an aqueous solution of phosphoric acid and then treated with a bonding agent containing acidic monomer. After filling, the composition is irradiated with visible light for curing.

The invention is now described with reference to the following examples.

## Comparative Example 1

A monomer liquid was prepared from 70 parts by weight of 2,2-bis[4-(3-methacryloyloxy-2-hydroxypropoxy)phenyl]-propane (referred to as bis-GMA hereinafter), 15 parts by weight of triethyleneglycol dimethacrylate (referred to as 3G hereinafter), and 15 parts by weight of neopentylglycol dimethacrylate (referred to as NPG hereinafter). To this monomer liquid was added 1 wt% of camphorquinone and an aldehyde as shown in Table 1. The resulting composition was placed in a cylindrical glass container (1 ml in capacity and 10 mm in diameter) and irradiated with visible light to examine the curing time. The light source was a slide projector (Model HILUX—H130, made by Rikagaku Seiki Co., Ltd.) equipped with a halogen tungsten lamp (1 kW). Two cutoff filters (2E filter of Eastman Kodak Company) were placed on the lens to remove ultraviolet rays (415 nm). The glass container was placed 20 cm away from the lens and the light was directed upward to the bottom of the container. The results are shown in Table 1—1.

TABLE 1—1

| No. | Aldehyde | Amount (mg) | Monomer* liquid (mg) | Curing time (sec) |
|---|---|---|---|---|
| 1 | Control | — | 226.0 | 240 |
| 2 | p-Tolualdehyde | 4.5 | 239.8 | 37 |
| 3 | p-n-Octylbenzaldehyde | 5.2 | 220.7 | 41 |
| 4 | p-Anisaldehyde | 4.4 | 235.4 | 42 |
| 5 | p-n-Hexyloxybenzaldehyde | 5.0 | 225.3 | 43 |
| 6 | o-Phthalaldehyde | 3.7 | 246.7 | 38 |
| 7 | Benzaldehyde | 3.7 | 233.5 | 45 |
| 8 | Lauraldehyde | 3.8 | 222.8 | 27 |
| 9 | Citronellal | 2.6 | 232.5 | 25 |
| 10 | 1,9-Nonanedial | 3.2 | 207.1 | 24 |
| 11 | Glutaric dialdehyde ** | 6.0 | 231.5 | 35 |

* Monomer liquid contains camphorquinone.
** 40% aqueous solution.

Comparative Example 2

Compositions were prepared by incorporating the monomer liquid obtained in Example 1 with 1 wt% of camphorquinone and various amines as shown in Table 1—2. The curing time was measured in the same way as in Example 1. The results are shown in Table 1—2.

TABLE 1—2

| No. | Amine | Amount (mg) | Monomer* liquid (mg) | Curing time (sec) |
|---|---|---|---|---|
| 1 | N,N-dimethylaminoethyl methacrylate | 5.1 | 295.0 | 55 |
| 2 | Triethylamine | 4.7 | 230.1 | 90 |
| 3 | N,N-dimethylethanolamine | 4.1 | 221.7 | 110 |
| 4 | n-Butylamine | 4.0 | 247.6 | 110 |
| 5 | N,N-diethanol-p-toluidine | 5.0 | 230.3 | 120 |

* Monomer liquid contains camphorquinone.

Example 1

Compositions were prepared by adding to the monomer liquid obtained in Comparative Example 1, camphorquinone (1 wt%), tolualdehyde (1.1 wt%), and various organic peroxides as shown in Table 2. The curing time was measured in the same way as in Example 1. The results are shown in Table 2.

TABLE 2

| No. | Peroxide | Amount (mg) | Monomer* liquid (mg) | Curing time (sec) |
|---|---|---|---|---|
| 1 | Control | — | 233.0 | 47 |
| 2 | Benzoyl peroxide | 2.3 | 246.0 | 30 |
| 3 | t-Butylperbenzoate | 5.5 | 220.8 | 33 |
| 4 | di-t-Butylperoxyisophthalate | 4.6 | 227.2 | 32 |
| 5 | 2,5-dimethyl-2,5-di(benzoil-peroxy)hexane | 2.3 | 218.5 | 29 |

* Monomer liquid contains camphorquinone and tolualdehyde.

Example 2 and Comparative Example 3

A monomer liquid composed of 33 parts by weight of U—4TH, 33 parts by weight of 2,2′-di(4-methacryloxypolyethoxyphenyl)propane (having 2 to 3 (2.6 on average) ethoxy groups per molecule), and 33 parts by weight of 3G was mixed with 0.95 wt% of camphorquinone. The monomer liquid was made into compositions by adding t-butyl perbenzoate and lauraldehyde as shown in Table 3. The resulting compositions were examined for curing time in the same way as in Example 1. The compositions for comparison did not include lauraldehyde. The results are shown in Table 3.

TABLE 3

| No. | Monomer liquid containing camphorquinone (mg) | Amount of lauraldehyde added (mg) | t-Butyl perbenzoate added (mg) | Curing time (sec) |
|---|---|---|---|---|
| 1* | 252.0 | — | — | 140—150 |
| 1* | 242.6 | 1.9 | — | 46 |
| 2* | 240.9 | — | 9.0 | 80 |
| 2 | 240.8 | 1.8 | 8.6 | 31 |

* Comparative examples.

Example 3 and Comparative Example 3A

The same monomer liquid as prepared in Example 1 was mixed with 0.97 wt% of camphorquinone and 1.03 wt% of benzoyl peroxide. This monomer liquid included aldehyde as shown in Table 4. The resulting compositions were examined for curing time in the same way as in Example 1. The results are shown in Table 4. Composition 1 is a Comparative Example.

TABLE 4

| No. | Monomer liquid containing quinone & BPO (mg) | Aldehyde | Amount (mg) | Curing time (sec) |
|---|---|---|---|---|
| 1 | 237.2 | — | — | 70 |
| 2 | 219.3 | p-Tolualdehyde | 4.2 | 28 |
| 3 | 217.7 | Lauraldehyde | 5.4 | 18 |
| 4 | 230.6 | 1,9-Nonanedial | 2.8 | 15 |
| 5 | 232.3 | p-Phthaldialdehyde | 4.8 | 28 |
| 6 | 235.6 | β-Naphthaldehyde | 1.9 | 42 |
| 7 | 226.3 | p-Cyanobenzaldehyde | 3.8 | 42 |
| 8 | 245.5 | Citral | 4.5 | 33 |
| 9 | 228.0 | Citronellal | 3.0 | 20 |

Comparative Example 4

The same monomer liquid as in comparative Example 1 was incorporated with a photosensitizer as shown in Table 5. The monomer liquid was divided into two portions. One portion was irradiated with light without addition of aldehyde and the other was irradiated with light after addition of p-tolualdehyde. They were examined for curing time. Irradiation was carried out in the same way as in Example 1. The results are shown in Table 5.

TABLE 5

| No. | Photosensitizer, amount added (wt%) | | Amount of monomer liquid (mg) | Amount of p-tolualdehyde added (mg) | Curing time (sec) |
|---|---|---|---|---|---|
| 1 | Diacetyl | (0.97) | 233.0 | — | 150 |
| 1 | Diacetyl | (0.97) | 232.6 | 6.6 | 120 |
| 2 | 2,3-Heptanedione | (1.09) | 234.9 | — | 240 |
| 2 | 2,3-Heptanedione | (1.09) | 234.1 | 3.5 | 210 |

Comparative Example 5

Comparative Example 1 was repeated using the compositions shown in Table 6. The quantity of the composition was 220 to 230 mg.

TABLE 6

| | Monomer composition (parts by weight) | | Sensitizer and amount (parts by weight) | | Accelerator and amount (parts by weight) | | Curing time (min) |
|---|---|---|---|---|---|---|---|
| 1* | Styrene<br>Pentaerythritol trimethacrylate | 25.8<br>74.2 | Camphorquinone | 0.99 | — | | 8 (gelation) |
| 1 | Styrene<br>Pentaerythritol trimethacrylate | 25.8<br>74.2 | Camphorquinone | 0.99 | Lauraldehyde | 2.5 | 2.5 |
| 2* | Epoxy ester (3002) †<br>Trimethylolpropane trimethacrylate<br>Methyl methacrylate | 33<br>33<br>33 | Camphorquinone | 0.99 | — | | 12< (no cure) |
| 2 | Epoxy ester (3002) †<br>Trimethylolpropane trimethacrylate<br>Methyl methacrylate | 33<br>33<br>33 | Camphorquinone | 0.99 | Lauraldehyde | 2.5 | 12 |
| 3* | Styrene<br>bis-GMA | 63<br>37 | Camphorquinone | 0.91 | — | | 10< (no cure) |
| 3 | Styrene<br>bis-GMA | 63<br>37 | Camphorquinone | 0.91 | Lauraldehyde | 2.5 | 9 |
| 4* | Epoxy ester (3002)<br>Methyl cinnamate | 79<br>21 | 2,3-Pentanedione | 1.62 | — | | 10< (no cure) |
| 4 | Epoxy ester (3002)<br>Methyl cinnamate | 79<br>21 | 2,3-Pentanedione | 1.62 | p-tolualdehyde | 2.5 | 10 |
| 5* | Phenyl P monomer ††<br>bis-GMA<br>Triethyleneglycol dimethacrylate | 5<br>75<br>20 | Camphorquinone | 0.73 | — | | 7 |
| 5 | Phenyl P monomer ††<br>bis-GMA<br>Triethyleneglycol dimethacrylate | 5<br>75<br>20 | Camphorquinone | 0.73 | Citral | 2.39 | 1.67 |
| 6 | Phenyl P monomer ††<br>bis-GMA<br>Triethyleneglycol dimethacrylate | 5<br>75<br>20 | Camphorquinone | 0.73 | Citronellal | 2.01 | 0.75 |

Note to Table 6.
* Comparative Examples.
† Structural formula:

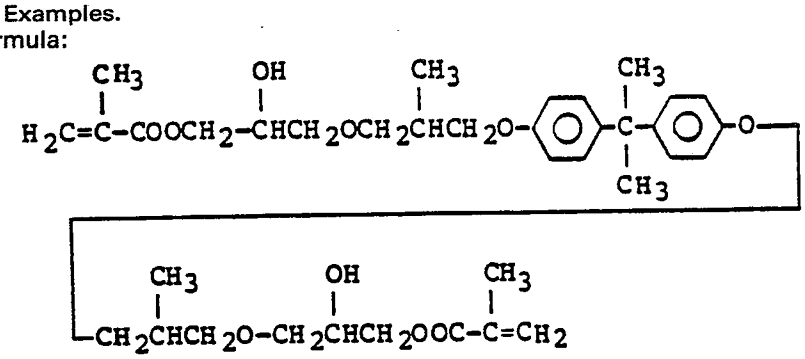

†† Structural formula:

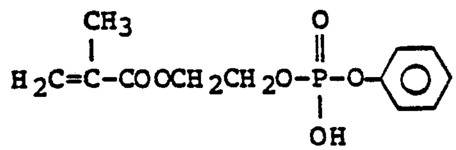

Example 4 and Comparative Example 6

A photopolymerizable dental composite resin containing aldehyde was prepared according to the following formulation (Example 4).

Formulation:

| | |
|---|---|
| Bis-GMA | 0.864 g |
| 3G | 0.288 g |
| Camphorquinone | 0.012 g |
| Benzoyl peroxide | 0.012 g |
| Lauraldehyde | 0.024 g |
| Silane-treated quartz powder | 3.871 g |
| Colloidal silica | 0.103 g |

These components were mixed in a mortar, and the resulting paste was degassed under vacuum. The paste sample was filled in a glass tube having an inside diameter of 4 mm, and was cured by irradiating with light emitted from a Translux (15V—150W halogen-tungsten lamp, made by Kulzer & Co., GMBH) placed 4 mm away. The light was directed to one end of the glass tube. From the other end of the glass tube a needle, 1 mm in diameter, was thrusted into the cured composition. A load of 260 g was applied to this needle for 30 seconds to measure the degree of penetration. This value was used to calculate the thickness of the cured resin. It was found that irradiation for 5 seconds and 10 seconds cured up to 11 mm and 20 mm, respectively.

In Comparative Example 6, the composition was prepared without lauraldehyde. The depth of cure by irradiation for 10 seconds was only 5 mm.

Example 5

A photopolymerizable dental composite resin was prepared according to the following formulation.

Formulation:

| | |
|---|---|
| Bis-GMA | 1.00 g |
| 2,2'-di(4-methacryloxypolyethoxyphenyl)propane (2.6 ethoxy groups on average in one molecule) | 1.00 g |
| 3G | 0.828 g |
| Camphorquinone | 4.6 mg |
| Benzoyl peroxide | 30.9 mg |
| p-Tolualdehyde | 85 mg |
| Titanium dioxide | 3.0 mg |
| Iron oxide red | 50 µg |
| Iron oxide yellow | 100 µg |
| Carbon black | 5 µg |
| Silane-treated quartz powder | 9.90 g |
| Colloidal silica | 0.25 g |

These components were mixed in a mortar to give a paste and the cure depth was measured in the same way as in Example 4.

Cure depths of 6.3 mm and 10.2 mm were accomplished with irradiation for 20 seconds and 60 seconds, respectively.

Comparative Example 7

A photopolymerizable composition was prepared according to the following formulation.

Formulation:

| | |
|---|---|
| Bis-GMA | 0.722 g |
| 3G | 0.309 g |
| Benzil | 0.02 g |
| Dimethylamino-benzaldehyde | 0.019 g |
| Silane-treated quartz powder | 3.379 g |
| Colloidal silica | 0.102 g |

These components were mixed in a mortar to give a paste and the cure depth was measured in the same way as in Example 4. A cure depth of 4.5 mm was achieved with irradiation for 20 seconds; but the cured product was severely colored yellow. It was apparently unsuitable for dental use. It is considered that this discoloration is due to dimethylaminobenzaldehyde used as a polymerization initiator.

On the other hand, no discoloration was observed in the cured resin compositions in Examples 4 and 5.

Example 6 and Comparative Example 8

Photopolymerizable compositions were prepared according to the formulations shown in Table 7. They were examined for storage stability. The results are shown in Table 8.

TABLE 7

| Component | Experiment No. 1* | 2* | 3* | 4* | 1 |
|---|---|---|---|---|---|
| Bis-GMA (g) ** | 0.7 | 0.7 | 0.7 | 0.7 | 0.7 |
| 3G (g) ** | 0.3 | 0.3 | 0.3 | 0.3 | 0.3 |
| Camphorquinone (g) | 0.005 | 0.005 | 0.005 | 0.005 | 0.005 |
| Accelerator (g) | | | | | |
| N,N-dimethylamino-benzaldehyde | 0.02 | 0.02 | — | — | — |
| N,N-dimethylamino-ethyl methacrylate | — | — | 0.02 | — | — |
| Terephthalaldehyde | — | — | — | 0.025 | 0.025 |
| BPO (g) | — | 0.01 | 0.01 | — | 0.01 |
| Silane-treated quartz powder (g) | 3.40 | 3.40 | 3.40 | 3.40 | 3.40 |
| Colloidal silica (g) | 0.10 | 0.10 | 0.10 | 0.10 | 0.10 |

* Comparative Examples

** Contains 500 ppm of 2,6-di-t-butyl-4-methylphenol.

TABLE 8

| No. | Number of days required for the composition to solidify at 50°C (days) | Number of days required for the composition to solidify at room temperature (days) |
|---|---|---|
| 1* | 14 | 60 |
| 2* | — | Solidified during preparation |
| 3* | 12 | 60 |
| 4* | >30 | >600 |
| 1 | 26 | >600 |

* Comparative Examples

It is to be noted from the above table that the composition of this invention has good storage stability.

Example 7 and Comparative Example 9

Dental composited resins were prepared according to the following formulations.

Formulation 1:

| | |
|---|---|
| 2,2'-di-(4-methacryloxy-polyethoxyphenyl)propane (having 2.6 ethoxy groups on average in one molecule) | 2.1 g |
| 3G | 0.9 g |
| Camphorquinone | 0.015 g |
| Benzoyl peroxide | 0.06 g |
| Tetradecyl aldehyde | 0.09 g |
| Silane-treated quartz powder | 11.7 g |
| Colloidal silica | 0.46 g |
| Iron oxide red | 50 μg |
| Iron oxide yellow | 100 μg |
| Titanium dioxide | 3 mg |

Formulation 2:

| | |
|---|---|
| Bis-GMA | 2.1 g |
| 3G | 0.9 g |
| Camphorquinone | 0.015 g |
| Benzoyl peroxide | 0.06 g |
| 3,4-Diethoxybenzaldehyde | 0.09 g |
| Silane-treated quartz powder | 11.7 g |
| Colloidal silica | 0.46 g |
| Iron oxide red | 50 μg |
| Iron oxide yellow | 100 μg |
| Titanium dioxide | 3 mg |

Formulation 3:

| | |
|---|---|
| Bis-GMA | 2.1 g |
| 3G | 0.9 g |
| Camphorquinone | 0.015 g |
| Benzoyl peroxide | 0.06 g |
| p-n-Octyloxybenzaldehyde | 0.09 g |
| Silane-treated quartz powder | 11.7 g |
| Colloidal silica | 0.46 g |
| Iron oxide red | 50 µg |
| Iron oxide yellow | 100 µg |
| Titanium dioxide | 3 mg |

Formulation 4: (Comparative)

| | |
|---|---|
| Bis-GMA | 2.1 g |
| 3G | 0.9 g |
| Camphorquinone | 0.015 g |
| N,N-dimethylaminoethyl methacrylate | 0.03 g |
| Silane-treated quartz powder | 11.7 g |
| Colloidal silica | 0.46 g |
| Iron oxide red | 50 µg |
| Iron oxide yellow | 100 µg |
| Titanium dioxide | 3 mg |

Formulation 5: (Comparative)

| | |
|---|---|
| Bis-GMA | 2.1 g |
| 3G | 0.9 g |
| Camphorquinone | 0.015 g |
| N,N-dimethylaminoethyl methacrylate | 0.03 g |
| Benzoyl peroxide | 0.06 g |
| Silane-treated quartz powder | 11.7 g |
| Colloidal silica | 0.46 g |
| Iron oxide red | 50 µg |
| Iron oxide yellow | 100 µg |
| Titanium dioxide | 3 mg |

The components of each formulation were mixed in a mortar and the resulting paste was degassed under vacuum. The thus prepared compositions were tested as follows:

(A) Each composition in the form of paste prepared as mentioned above was filled into a cylindrical cavity, 4 mm in diameter and 3 mm deep, formed on the occusal surface of a human molar. Prior to filling, the cavity was treated with 40% phosphoric acid and then a bonding agent ("Clearfil New Bond") containing a phosphate ester monomer. The filled paste was irradiated for 40 seconds with light emitted from Translux. After curing, the sample molar was dipped in water at 37°C for 24 hours. Excess of resin was ground off. The sample molar was dipped in cold water (4°C) and hot water (60°C) for 1 minute each alternately. The dipping was repeated 100 times. Finally, the sample molar was dyed with 0.1% aqueous solution of basic fuchsin. There was no sign of fuchsin entering the tooth cavities in formulations 1 to 3. But there was a little in formulations 4 to 5.

(B) The composition was examined for adhesive strength. The bovine anterior labial surface was polished with emery paper to make a smooth enamel surface. A piece of adhesive tape having a hole, 5 mm in diameter, was attached to the smooth enamel surface to establish the bonding area. The enamel surface was treated with 40% phosphoric acid for 45 seconds (acid etching) and then a commercial bonding agent ("Clearfil New Bond") was applied to the etched surface. The paste prepared as described above was placed on the treated enamel surface and spread over it with a piece of glass. The paste was cured by irradiating for 40 seconds with light emitted from a Translux. After curing, the glass was removed. A stainless steel round rod (7 mm in diameter) was bonded vertically to the cured resin with a commercial adhesive filling material ("Clearfil F II"). The assembly was dipped in water at 37°C overnight. The adhesive strength was measured by using an Instron TT—B universal materials testing machine. The above-mentioned test was also conducted for the human tooth dentin. The results are shown in Table 9.

TABLE 9

| | Bovine enamel ($kg/cm^2$) | Human dentin ($kg/cm^2$) |
|---|---|---|
| Example 7 | | |
| Formulation 1 | 170 | 140 |
| Formulation 2 | 170 | 130 |
| Formulation 3 | 160 | 130 |
| Comparative Example 9 | | |
| Formulation 4 | 92 | 80 |
| Formulation 5 | 130 | 95 |

(C) The composition was examined for compressive strength. The paste prepared as described above was placed in a stainless steel cylindrical mold, 4 mm high and 4 mm in diameter, and compacted by glass plates at both ends of the mold. The paste in the mold was irradiated for one minute each for top and bottom with a Translux. After curing, the assembly was kept in a constant temperature air bath at 37°C for 24 hours. The cured sample was removed from the mold, and the compressive strength of the sample was measured by using an Instron tester.

The paste prepared as described above was also placed in a stainless steel cylindrical mold, 3 mm high and 6 mm in diameter, and cured in the same way as described above. The cured sample was removed from the mold and dipped in water at 37°C for 24 hours. The diametral tensile strength of the sample was measured by using an Instron tester. The results are shown in Table 10.

TABLE 10

| | Compressive strength (kg/cm²) | Diametral tensile strength (kg/cm²) |
|---|---|---|
| Example 7 | | |
| Formulation 1 | 3500 | 580 |
| Formulation 2 | 3400 | 600 |
| Formulation 3 | 3450 | 610 |
| Comparative Example 9 | | |
| Formulation 4 | 2820 | 530 |
| Formulation 5 | 3090 | 530 |

(D) The compositions were examined for storge stability as follows: The pastes of Formulation 2 and Formulation 5 (Comparative Example) were stored in a constant temperature air bath at 45°C. At regular intervals, the samples were examined for compressive strength, diametral tensile strength, and cure depth to see the change in performance with time. The samples for compressive strength and diametral tensile strength were irradiated for 1 minute with Translux as shown in Example 12(C) and the samples for cure depth were irradiated for 20 seconds. The results are shown in Table 11.

TABLE 11

| Period of storage (days) | | 0 | 3 | 7 | 14 | 21 |
|---|---|---|---|---|---|---|
| Formulation 2 | | | | | | |
| Compressive strength | (kg/cm²) | 3400 | 3460 | 3475 | 3520 | 3460 |
| Tensile strength | (kg/cm²) | 600 | 632 | 649 | 648 | 640 |
| Depth of cure | (mm) | 5.00 | 4.81 | 4.67 | 5.04 | 5.14 |
| Formulation 5* | | | | | | |
| Compressive strength | (kg/cm²) | 3090 | 3120 | 3130 | 2920 | 3150 |
| Tensile strength | (kg/cm²) | 530 | 545 | 470 | 540 | 540 |
| Depth of cure | (mm) | 5.10 | 4.63 | 3.93 | 3.85 | 3.49 |

*The samples were tinged with red under the above storage conditions.

## Claims

1. A photopolymerizable composition composed of a polymerizable monomer and an initiator capable of polymerizing the monomer upon exposure to visible light, characterized in that the initiator consists essentially of (a) at least one photosensitizer that is an aliphatic or cycloaliphatic α-diketone, (b) at least one accelerator selected from aldehydes containing no amino groups and (c) an organic peroxide.

2. A photopolymerizable composition as claimed in claim 1, in which the α-diketone is a compound represented by the formula:

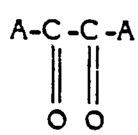

where each A denotes an aliphatic hydrocarbon group of 1 to 20 carbon atoms, which may be the same as or different from the other A, or the two As are bonded to one another to form a cycloaliphatic structure.

3. A composition as claimed in claim 2, in which the photosensitizer is one or more of camphorquinone, diacetyl, 2,3-pentanedione, 2,3- or 3,4-hexanedione, 2,3-, 3,4- or 4,5-octanedione.

4. A composition as claimed in any one of claims 1 to 3, in which the aldehyde is a compound represented by the formula $B{+}CHO)_n$, where *n* is 1, 2 or 3 and B is an acyclic or alicyclic saturated or unsaturated aliphatic hydrocarbon group of 1 to 20 carbon atoms or a monocyclic or polycyclic aromatic hydrocarbon group of 1 to 20 carbon atoms, optionally having a $C_{1-20}$ substituent.

5. A composition as claimed in claim 4, in which the aldehyde is a $C_{1-20}$ alkylmonoaldehyde or alkyldialdehyde, a $C_{1-20}$ polyalkyl ether mono- or dialdehyde, or a compound represented by the formula:

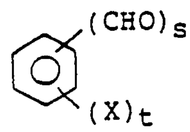

where s is 1 or 2; X is a $C_{1-20}$ alkyl, alkoxy or polyalkylether group; and t is 0, 1, 2 or 3.

6. A composition as claimed in any one of claims 1 to 5, in which the organic peroxide is a diacyl peroxide or peroxy ester.

7. A composition as claimed in claim 6, in which the organic peroxide benzoyl peroxide, *t*-butyl perbenzoate, di-*t*-butyl peroxyisophthalate, or 2,5-dimethyl-2,5-di(benzoylperoxy)hexane.

8. A composition as claimed in any one of claims 1 to 7, that further comprises a filler.

9. A composition as claimed in claim 8, for use as a dental filling material.

**Patentansprüche**

1. Photopolymerisierbare Masse aus einem polymerisierbaren Monomeren und einem Initiator, der dazu in der Lage ist, das Monomere bei der Einwirkung von sichtbarem Licht zu polymerisieren, dadurch gekennzeichnet, daß der Initiator im wesentlichen aus (a) wenigstens einem Photosensibilisator, der ein aliphatisches oder cycloaliphatisches α-Diketon ist, (b) wenigstens einem Beschleuniger, ausgewählt aus Aldehyden, die keine Aminogruppen enthalten, und (c) einem organischen Peroxid besteht.

2. Photopolymerisierbare Masse nach Anspruch 1, in welcher das α-Diketon eine Verbindung der Formel

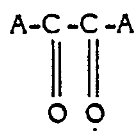

ist, worin jeweils A eine aliphatische Kohlenwasserstoffgruppe mit 1 bis 20 Kohlenstoffatomen ist, welche gleich dem anderen A oder davon verschieden sein kann, oder die zwei A miteinander unter Bildung einer cycloaliphatischen Struktur verbunden sind.

3. Masse nach Anspruch 2, in welcher der Photosensibilisator aus einer oder mehreren der Verbindungen Kampferchinon, Diacetyl, 2,3-Pentandion, 2,3- oder 3,4-Hexandion oder 2,3-, 3,4- oder 4,5-Oktandion besteht.

4. Masse nach einem der Ansprüche 1 bis 3, in welcher der Aldehyd eine Verbindung der Formel $B{+}CHO)_n$ ist, worin n 1, 2 oder 3 ist und B eine acyclische oder alicyclische gesättigte oder ungesättigte aliphatische Kohlenwasserstoffgruppe mit 1 bis 20 Kohlenstoffatomen oder eine monocyclische oder polycyclische aromatische Kohlenwasserstoffgruppe mit 1 bis 20 Kohlenstoffatomen, gegebenenfalls mit einem $C_{1-20}$-Substituenten, ist.

5. Masse nach Anspruch 4, in welcher der Aldehyd ein $C_{1-20}$-Alkylmonoaldehyd oder -alkyldialdehyd, ein $C_{1-20}$-Polyalkylethermono- oder -dialdehyd oder eine Verbindung der Formel

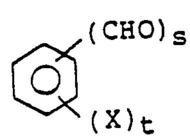

ist, worin s 1 oder 2 ist; X eine $C_{1-20}$-Alkyl, -Alkoxy oder -Polyalkylethergruppe ist und t 0, 1, 2 oder 3 ist.

6. Masse nach einem der Ansprüche 1 bis 5, in welcher das organische Peroxid ein Diacylperoxid oder Peroxyester ist.

7. Masse nach Anspruch 6, in welcher das organische Peroxid aus Benzoylperoxid, t-Butylperbenzoat, Di-t-butylperoxyisophthalat oder 2,5-Dimethyl-2,5-di(benzoylperoxy)hexan besteht.

8. Masse nach einem der Ansprüche 1 bis 7, die ausserdem einen Füllstoff enthält.

9. Masse nach Anspruch 8 für eine Verwendung als Zahnfüll-material.

**Revendications**

1. Une composition photopolymérisable composée d'un monomère polymérisable et d'un amorceur capable de polymériser le monomère par exposition à la lumière visible, caractérisée en ce que l'amorceur est constitué essentiellement de (a) au moins un photosensibilisateur qui est une α-dicétone aliphatique ou cycloaliphatique, (b) au moins un accélérateur choisi parmi les aldéhydes ne contenant pas de groupes amino et (c) un peroxyde organique.

2. Une composition photopolymérisable selon la revendication 1, dans laquelle l'α-dicétone est un composé représenté par la formule:

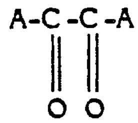

dans laquelle chaque A représente un groupe hydrocarboné aliphatique de 1 à 20 atomes de carbone, qui peut être semblable ou différent de l'autre A, ou les deux A sont unis entre eux pour former une structure cycloaliphatique.

3. Une composition selon la revendication 2, dans laquelle le photosensibilisateur est un ou plusieurs de la camphoquinone, du diacétyle, de la 2,3-pentanedione, de la 2,3- ou 3,4-hexanedione ou de la 2,3-, 3,4- ou 4,5-octanedione.

4. Une composition selon l'une quelconque des revendications 1 à 3, dans laquelle l'aldéhyde est un composé représenté par la formule $B\text{-}(CHO)_n$, dans laquelle n est 1, 2 ou 3 et B est un groupe hydrocarboné aliphatique acyclique ou alicyclique saturé ou insaturé de 1 à 20 atomes de carbone ou un groupe hydrocarboné aromatique monocyclique ou polycyclique de 1 à 20 atomes de carbone, ayant facultativement un substituant en $C_{1-20}$.

5. Une composition selon la revendication 4, dans laquelle l'aldéhyde est un (alcoyl en $C_{1-20}$) mono-aldéhyde ou (alcoyl en $C_{1-20}$) dialdéhyde, un poly(alcoyl en $C_{1-20}$éther)-mono- ou -dialdéhyde ou un composé représenté par la formule:

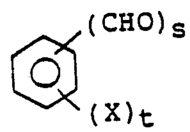

dans laquelle s est 1 ou 2; X est un groupe alcoyle en $C_{1-20}$, alcoxy ou polyalcoyléther; et t est 0, 1, 2 ou 3.

6. Une composition selon l'une quelconque des revendications 1 à 5, dans laquelle le peroxyde organique est un peroxyde de diacyle ou un peroxyester.

7. Une composition selon la revendication 6, dans laquelle le peroxyde organique est le peroxyde de benzoyle, le perbenzoate de tert-butyle, le peroxyisophtalate de di-tert-butyle ou le 2,5-diméthyl-2,5-di(benzoylperoxy)hexane.

8. Une composition selon l'une quelconque des revendications 1 à 7, qui comprend de plus une charge.

9. Une composition selon la revendication 8 pour l'emploi comme matériau d'obturation dentaire.